(19) Europäisches Patentamt
European Patent Office
Office européen des brevets

(11) Veröffentlichungsnummer: **0 224 219 B1**

(12) **EUROPÄISCHE PATENTSCHRIFT**

(45) Veröffentlichungstag der Patentschrift: **15.05.91**

(51) Int. Cl.[5]: **C07C 275/30**, A01N 47/34

(21) Anmeldenummer: **86116187.5**

(22) Anmeldetag: **22.11.86**

# (54) N-Benzoyl-,N'-phenoxyphenylharnstoffe, Verfahren zu ihrer Herstellung und ihre Verwendung zur Bekämpfung von Schädlingen.

(30) Priorität: **29.11.85 DE 3542201**

(43) Veröffentlichungstag der Anmeldung:
**03.06.87 Patentblatt 87/23**

(45) Bekanntmachung des Hinweises auf die Patenterteilung:
**15.05.91 Patentblatt 91/20**

(84) Benannte Vertragsstaaten:
**AT BE CH DE FR GB IT LI**

(56) Entgegenhaltungen:
**EP-A- 0 044 410**
**EP-A- 0 057 888**
**EP-A- 0 074 074**
**EP-A- 0 093 976**
**EP-A- 0 093 977**
**EP-A- 0 101 990**
**EP-A- 0 140 590**
**EP-A- 0 161 019**
**WO-A-86/05780**
**DE-A- 2 531 202**
**DE-A- 2 537 413**

**PATENT ABSTRACTS OF JAPAN, unexamined applications, C Feld, Band 4, no. 4, 28. Mai 1980, THE PATENT OFFICE JAPANESE GOVERNMENT, p. 40 C 12**

(73) Patentinhaber: **BASF Aktiengesellschaft**
**Carl-Bosch-Strasse 38**
**W-6700 Ludwigshafen(DE)**

(72) Erfinder: **Lange, Arno, Dr.**
**Oberes Geistal 3 b**
**W-6702 Bad Dürkheim(DE)**
Erfinder: **Seppelt, Wolfgang, Dr.**
**Lucas-Cranach-Strasse 8**
**W-6712 Bobenheim-Roxheim(DE)**
Erfinder: **Adolphi, Heinrich, Dr.**
**Kalmitweg 11**
**W-6703 Limburgerhof(DE)**

**EP 0 224 219 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue N-Benzoyl-,N′-phenoxyphenylharnstoffe, Verfahren zu ihrer Herstellung und Schädlingsbekämpfungsmittel, die diese Verbindungen als Wirkstoffe enthalten.

Es ist bekannt, daß N-Benzoyl-,N′-phenylharnstoffe als Insektizide verwendet werden können (J. Agr. Food Chem. 21, 348 (1973);
DE-OS 25 31 202).

Auch viele N-Benzoyl-,N′-phenoxyphenylharnstoffe sind bekannt, wozu auf die nachstehenden Druckschriften verwiesen wird.

Daher könnte man erwarten, daß Verbindungen aus
US-PS 4 399 152; DE-OS 25 31 202 (≙ US 4 068 002); EP 101 990;
JA 80-38 357; EP 140 590; DE-OS 25 37 413 (≙ US 4 005 223) oder
DE-OS 3 309 987, sämtlich Wirkstoffe, die eine biologisch ähnliche Wirkung wie die erfindungsgemäßen Verbindungen entfalten, eine erschöpfend untersuchte Stoffklasse darstellen.

Es überraschte daher, daß spezielle N-Benzoyl-,N′-phenoxyphenylharnstoffe der Formel

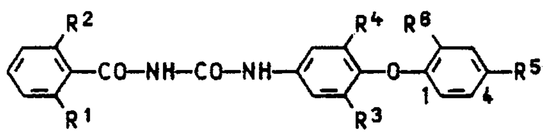

(I),

in der
$R^1$ Fluor oder Chlor,
$R^2$ Fluor, Chlor (oder Wasserstoff, wenn $R^1$ Chlor ist),
$R^3$ und $R^4$ unabhängig Chlor oder Brom und
$R^5$ und $R^6$ unabhängig Fluor, Chlor oder Brom bedeuten
den chemisch am nächsten kommenden bekannten Stoffen, als die die der JA-OS 80-38 357 angesehen werden, deutlich überlegen sind. Insbesondere können Schädlinge besser bekämpft werden, die wirtschaftlich von besonderer Bedeutung sind.

Dies überrascht ganz besonders, da die erfindungsgemäß erforderliche Substitution (z.B. 2,4-Dichlorphenoxy) unter wirtschaftlichen Gesichtspunkten günstiger ist als bekannte wie 3,5-Difluorphenoxy; 2-Chlor, 4-Trifluormethoxyphenoxy oder 2-Chlor-, 4-Trifluormethylphenoxy. Besonders wirtschaftlich sind Verbindungen, bei denen $R^3 = R^4 = Cl$ und/oder $R^5 = R^6 = Cl$ oder Br ist.

Besonders wirksam sind Verbindungen, bei denen $R^6 = F$ und $R^5 = Br$ oder Cl ist.

Für den Benzoylteil gilt, daß der 2,6-Difluor-benzoylrest wegen der biologischen Wirkung bevorzugt ist. Am preisgünstigsten und ähnlich wirksam ist der 2-Chlorbenzoylrest.

Man erhält die N-Benzyl-,N′-phenoxyphenylharnstoffe in bekannter Weise durch Umsetzung von entsprechenden Anilinen und Benzoylisocyanaten oder von Phenoxyphenylisocyanaten mit Benzamiden, die durch die Formeln

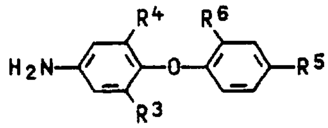

(II),

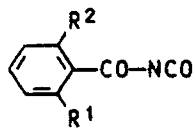

(III),

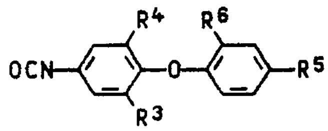

(IV)

bzw.

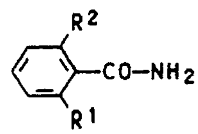

(V)

dargestellt werden und entweder bekannt sind oder mit fachüblichen Mitteln erhältlich sind.

Die Herstellungsverfahren entsprechen den in den vorstehenden Druckschriften beschriebenen, auf die deshalb verwiesen wird.

Herstellungsbeispiel

1) 5 g 3,5-Dichlor-4-(2,4-dichlorphenoxy)anilin werden in 100 ml Toluol gelöst und tropfenweise mit 3,2 g 2,6-Difluorbenzoylisocyanat in 10 ml Toluol versetzt. Nach 3 Stunden wird abgesaugt, mit wenig Toluol gewaschen und getrocknet. Ohne Berücksichtigung der Reste in der Waschflüssigkeit gewinnt man 5,9 g N-2,6-Difluorbenzoyl,N′-[3,5-dichlor,4-(2,4-dichlorphenoxy)phenyl]harnstoff vom Schmelzpunkt 209 bis 212 °C.

2) 5 g 3,5-Dichlor-4-(2,4-dichlorphenoxy)anilin werden in 100 ml Toluol gelöst und tropfenweise mit 3,2 g 2-Chlorbenzoylisocyanat in 10 ml Toluol versetzt. Nach 3 Stunden wird abgesaugt, mit Toluol gewaschen und getrocknet: 6,1 g N-2-Chlorbenzoyl,N′-[3,5-dichlor-4-(2,4-dichlorphenoxy)phenyl]harnstoff vom Schmelzpunkt 210 bis 212 °C.

Die von der allgemeinen Formel I umfaßten erfindungsgemäßen Verbindungen können z.B. die in der nachstehenden Tabelle aufgeführten speziellen Stoffe sein (die Bedeutungen von $R^1$ bis $R^6$ entsprechen der Beschreibung).

| Nr. | $R^1$ | $R^2$ | $R^3$ | $R^4$ | $R^5$ | $R^6$ | Schmelzpunkt (°C) |
|---|---|---|---|---|---|---|---|
| 1 | F | F | Cl | Cl | Cl | Cl | 209 - 212 |
| 2 | Cl | Cl | Cl | Cl | Cl | Cl | 253 - 255 |
| 3 | Cl | H | Cl | Cl | Cl | Cl | 210 - 212 |
| 4 | F | Cl | Cl | Cl | Cl | Cl | |
| 5 | F | F | Cl | Cl | F | Cl | 136 - 140 |
| 6 | Cl | Cl | Cl | Cl | F | Cl | |
| 7 | Cl | H | Cl | Cl | F | Cl | 128 - 131 |
| 8 | F | Cl | Cl | Cl | Cl | Cl | |
| 9 | F | Cl | Cl | Cl | Cl | F | |
| 10 | F | F | Cl | Cl | F | F | |
| 11 | Cl | Cl | Cl | Cl | F | F | |
| 12 | Cl | H | Cl | Cl | F | F | |
| 13 | F | F | Cl | Cl | Cl | F | 172 - 185 |
| 14 | Cl | Cl | Cl | Cl | Cl | F | |
| 15 | Cl | H | Cl | Cl | Cl | F | 192 - 196 |
| 16 | F | F | Cl | Cl | Br | Br | 199 - 202 |
| 17 | Cl | Cl | Cl | Cl | Br | Br | |
| 18 | F | F | Cl | Cl | Br | F | 199 - 202 |
| 19 | Cl | Cl | Cl | Cl | Br | F | |
| 20 | Cl | H | Cl | Cl | Br | F | 235 - 240 |
| 21 | F | F | Cl | Cl | Br | Cl | 200 - 204 |
| 22 | Cl | Cl | Cl | Cl | Br | Cl | 250 - 252 |
| 23 | Cl | H | Cl | Cl | Br | Cl | 210 - 211 |
| 24 | F | F | Cl | Cl | F | Br | 198 - 200 |
| 25 | Cl | Cl | Cl | Cl | F | Br | |
| 26 | Cl | H | Cl | Cl | F | Br | 237 - 242 |
| 27 | F | F | Cl | Cl | Cl | Br | 209 - 211 |
| 28 | Cl | Cl | Cl | Cl | Cl | Br | 257 - 260 |
| 29 | Cl | H | Cl | Cl | Cl | Br | 204 - 206 |
| 30 | F | F | Br | Cl | Cl | Cl | 194 - 197 |
| 31 | Cl | Cl | Br | Cl | Cl | Cl | |
| 32 | Cl | H | Br | Cl | Cl | Cl | 206 - 208 |

| Nr. | $R^1$ | $R^2$ | $R^3$ | $R^4$ | $R^5$ | $R^6$ | Schmelzpunkt (°C) |
|---|---|---|---|---|---|---|---|
| 33 | F | Cl | Br | Cl | Cl | Cl | |
| 34 | F | F | Br | Cl | F | Cl | |
| 35 | Cl | Cl | Br | Cl | F | Cl | |
| 36 | Cl | H | Br | Cl | F | Cl | |
| 37 | F | Cl | Br | Cl | F | Cl | |
| 38 | F | F | Br | Cl | F | F | |
| 39 | Cl | Cl | Br | Cl | F | F | |
| 40 | Cl | H | Br | Cl | F | F | |
| 41 | F | F | Br | Cl | Cl | F | |
| 42 | Cl | Cl | Br | Cl | Cl | F | |
| 43 | Cl | H | Br | Cl | Cl | F | |
| 44 | F | F | Br | Cl | Br | Br | |
| 45 | Cl | Cl | Br | Cl | Br | Br | |
| 46 | Cl | H | Br | Cl | Br | Br | |
| 47 | F | F | Br | Cl | Br | F | |
| 48 | Cl | Cl | Br | Cl | Br | F | |
| 49 | Cl | H | Br | Cl | Br | F | |
| 50 | F | F | Br | Cl | Br | Cl | |
| 51 | Cl | Cl | Br | Cl | Br | Cl | |
| 52 | Cl | H | Br | Cl | Br | Cl | |
| 53 | F | F | Br | Cl | F | Br | |
| 54 | Cl | Cl | Br | Cl | F | Br | |
| 55 | Cl | H | Br | Cl | F | Br | |
| 56 | F | F | Br | Cl | Cl | Br | |
| 57 | Cl | Cl | Br | Cl | Cl | Br | |
| 58 | Cl | H | Br | Cl | Cl | Br | |
| 59 | F | F | Br | Br | Cl | Cl | 204 - 207 |
| 60 | Cl | Cl | Br | Br | Cl | Cl | 263 - 268 |
| 61 | Cl | H | Br | Br | Cl | Cl | 225 - 227 |
| 62 | F | Cl | Br | Br | Cl | Cl | |
| 63 | F | F | Br | Br | F | Cl | |
| 64 | Cl | Cl | Br | Br | F | Cl | |
| 65 | Cl | H | Br | Br | F | Cl | |
| 66 | F | Cl | Br | Br | F | Cl | |
| 67 | F | F | Br | Br | F | F | |
| 68 | Cl | Cl | Br | Br | F | F | |
| 69 | Cl | H | Br | Br | F | F | |
| 70 | F | F | Br | Br | Cl | F | |
| 71 | Cl | Cl | Br | Br | Cl | F | |
| 72 | Cl | H | Br | Br | Cl | F | |
| 73 | F | F | Br | Br | Br | Br | |
| 74 | Cl | Cl | Br | Br | Br | Br | |

| Nr. | $R^1$ | $R^2$ | $R^3$ | $R^4$ | $R^5$ | $R^6$ | Schmelzpunkt (°C) |
|---|---|---|---|---|---|---|---|
| 75 | Cl | H | Br | Br | Br | Br | |
| 76 | F | F | Br | Br | Br | F | |
| 77 | Cl | Cl | Br | Br | Br | F | |
| 78 | Cl | H | Br | Br | Br | F | |
| 79 | F | F | Br | Br | Br | Cl | |
| 80 | Cl | Cl | Br | Br | Br | Cl | |
| 81 | Cl | H | Br | Br | Br | Cl | |
| 82 | F | F | Br | Br | F | Br | |
| 83 | Cl | Cl | Br | Br | F | Br | |
| 84 | Cl | H | Br | Br | F | Br | |
| 85 | F | F | Br | Br | Cl | Br | |
| 86 | Cl | Cl | Br | Br | Cl | Br | |
| 87 | Cl | H | Br | Br | Cl | Br | |
| 88 | Cl | H | Cl | Cl | Br | Br | 201 - 204 |
| 89 | F | Cl | Cl | Cl | Br | Br | |

Die N-Benzoyl-,N′-phenoxyphenylharnstoffe der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Als Vergleichsmittel wurden folgende Verbindungen gewählt:

| Vergleichsmittel | aus | Struktur |
|---|---|---|
| I | DE-OS 33 09 987 | 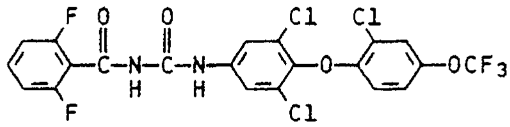 |
| II | EP 101 990 | 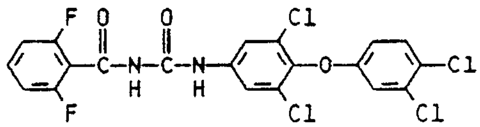 |
| III | EP 101 990 | 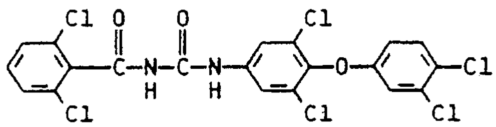 |
| IV | EP 101 990 | 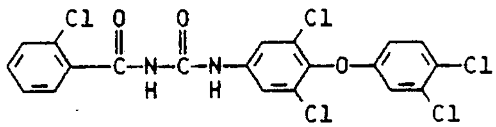 |
| V | US 40 68 002 | 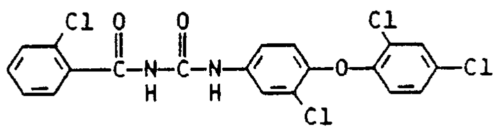 |
| VI | J 5 5038-357 | 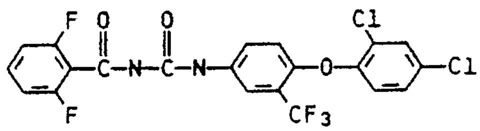 |

Zuchtversuch mit Stubenfliege (Musca domestica)

Als Versuchsgefäße dienen 50 ml Penicillingläser. Diese werden mit je 4,75 ml gekochter Magermilch beschickt und mit 0,25 ml wäßriger Wirkstoffaufbereitung versetzt. Jedem Glas fügt man ein vorgefertigtes Wattebällchen (Fa. Hartmann, Maintal) zu.

In einem Becherglas schwemmt man ca. 10 000 einen Tag alte Fliegeneier in ca. 100 ml Leitungswasser auf. Aus dieser Suspension überträgt man mittels einer Pipette je einen Tropfen in ein Glas.

Der fertige Versuchsansatz wird mit einem Filterpapier abgedeckt und bei 23 bis 24° C gelagert.

Am 4. Tag erfolgt die Auswertung. Beurteilt wird Entwicklungshemmung (+) und Mortalität (über 90 % +).

Ergebnisse:

Bei diesen Versuchen erzielten die Verbindungen 4, 10, 13, 21 bei einer Konzentration von 2 ppm eine bessere Wirkung als die Vergleichsmittel I, V, VI.

Zuchtversuch mit Schaben (Blatta orientalis)

Die Prüfung erfolgt in 1 l-Gläsern an 50 Schaben im 1. Larvenstadium. Ein Gestell aus 5 horizontalen Pappscheiben (6 x 6 cm) bietet den Tieren Schlupfmöglichkeiten.

Der Wirkstoff wird über das Futter aufgenommen. Dieses besteht aus 20 g kalt angerührtem Karottenbrei in Petrischalen (Ø 5 cm). Zusätzlich erhalten sie Wasser auf Zellstoff in Plastikbechern (Inhalt 25 ml). Die Beobachtungszeit erstreckt sich über zwei Häutungsphasen - 3 Wochen. Dabei muß wöchentlich mit frisch zubereitetem Brei nachgefüttert werden.

Die Beurteilung erfolgt ebenfalls wöchentlich nach:
a) % Mortalität
b) Entwicklungszustand
Bei diesem Versuch erzielten die Verbindungen 10, 13, 16, 18, 21, 24 bei einer Konzentration von 20 ppm eine bessere Wirkung als das Vergleichsmittel VI.

Zuchtversuch mit Baumwollwanzen (Dysdercus intermedius)

Baumwollwanzen Dysdercus intermedius werden im 4. Larvenstadium in Petrischalen (Ø 10 cm) dem Wirkstoffbelag der Testsubstanz für 24 Stunden ausgesetzt.
Die Überlebenden züchtet man in 1 l-Gläsern auf feuchtem Quarzsand, der vorher mit der Wirkstofflösung versetzt wurde bis zum Schlüpfen der $F_1$-Generation.
Dabei entsprechen in der Behandlung

| | |
|---|---|
| 2,5 mg/Schale | 25 ppm im Sand |
| 1,0 mg/Schale | 10 ppm im Sand |
| 0,5 mg/Schale | 5 ppm im Sand |

usw.
Beurteilt wird Mortalität und Vermehrung.
Bei diesem Versuch erzielten die Verbindungen 1, 3, 10, 13, 15, 16, 21, 23, 27, 29 bei einer Konzentration von 2 ppm eine bessere Wirkung als die Vergleichsmittel II, III, IV, V, VI.

Wirkung auf Larven der Mehlmotte (Ephestia kuehnielle), Zuchtversuch

Weizenmehl, welches stark mit Eiern der Mehlmotte belegt ist, wird mit den Wirkstoffen innig gemischt. 10 g werden in Joghurtgläser (250 ml) abgefüllt und bei 22° C gelagert.
Nach 4 Wochen beurteilt man die Entwicklung der Larven.
Bei diesem Versuch erzielten die Verbindungen 1, 3, 10, bei einer Konzentration von 2 ppm eine bessere Wirkung als die Vergleichsmittel IV, V, VI.

Zuchtversuch mit Moskito-Larven (Aedes aegypti)

200 ml Leitungswasser in 250 ml Plastikbechern werden mit der Wirkstoffaufbereitung versetzt. Darauf belegt man jeden Becher mit ca. 50 schlüpfreifen Eiern von Aedes aegypti, die auf der Papierunterlage haften, welche wir bei der Eiablage bieten.
Die Versuchstemperatur beträgt 25° C. Beurteilt wird Entwicklung und Schlupf der Imagines, wobei eine unbehandelte Kontrolle als Maßstab dient.
Während der Versuchsdauer wird nach Bedarf mit TETRAMIN gefüttert.
Bei diesem Versuch erzielten die Verbindungen 3 und 10 bei einer Konzentration von 0,004 ppm eine bessere Wirkung als die Vergleichsmittel III, IV, V.

Entwicklungshemmung bei Prodenia litura
(Prüfung auf behandeltem Nährboden)

100 g des Standard-Nährbodens für Prodenia werden in 250 ml-Becher gefüllt und warm mit der wäßrigen Wirkstoffaufbereitung sorgfältig gemischt.
Nach Erkalten belegt man jedes Gefäß mit 10 L 3-Raupen (1,5-1,8 cm) und lagert sie bei 23° C. Beurteilt wird Verpuppung und Schlupf der Falter.
Pro Konzentration müssen mindestens 2 Becher angesetzt werden.
Bei diesem Versuch erzielten die Verbindungen 10, 12, 18 bei einer Konzentration von 0,04 ppm eine bessere Wirkung als die Vergleichsmittel I und IV.

## Ansprüche

**1.** N-Benzoyl-,N'-phenoxyphenylharnstoffe der Formel

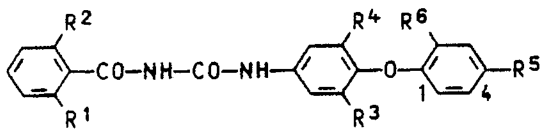

(I),

in der
$R^1$ Fluor oder Chlor
$R^2$ Fluor, Chlor (oder Wasserstoff, wenn $R^1$ Chlor ist)
$R^3$ und $R^4$ unabhängig Chlor oder Brom und
$R^5$ und $R^6$ unabhängig Fluor, Chlor oder Brom bedeuten.

**2.** Verfahren zur Herstellung von N-Benzoyl-,N'-phenoxyphenylharnstoffen der Formel I gemäß Anspruch 1, dadurch gekennzeichnet, daß man entweder eine entsprechende Verbindung der Formel

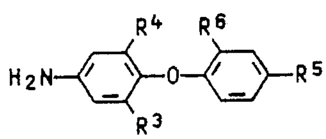

(II),

mit einem entsprechenden Benzoylisocyanat der Formel

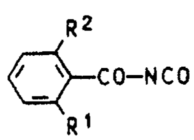

(III),

oder eine entsprechende Verbindung der Formel

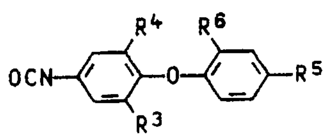

(IV)

mit einem entsprechenden Benzamid der Formel

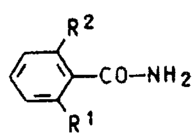

(V)

umsetzt.

**3.** Schädlingsbekämpfungsmittel, enthaltend einen N-Benzoyl-,N'-phenoxyphenylharnstoff der Formel I gemäß Anspruch 1.

**4.** Schädlingsbekämpfungsmittel, enthaltend einen festen oder flüssigen Trägerstoff und/oder einen Synergisten und einen N-Benzoyl-,N'-phenoxyphenylharnstoff der Formel I gemäß Anspruch 1.

**5.** Verwendung von N-Benzoyl-,N'-phenoxyphenylharnstoffen der Formel I gemäß Anspruch 1 zur Bekämpfung von Schädlingen.

6. Verfahren zur Bekämpfung von Schädlingen, dadurch gekennzeichnet, daß man eine wirksame Menge eines N-Benzoyl-,N′-phenoxyphenylharnstoffs der Formel I gemäß Anspruch 1 auf die Schädlinge und/oder deren Lebensraum einwirken läßt.

## Claims

1. An N-benzoyl-N'-phenoxyphenylurea of the formula

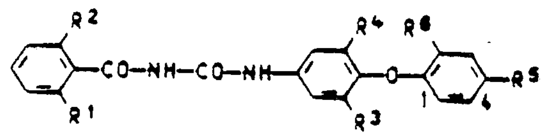

(I)

where $R^1$ is fluorine or chlorine, $R^2$ is fluorine or chlorine (or hydrogen if $R^1$ is chlorine), $R^3$ and $R^4$ independently of one another are each chlorine or bromine and $R^5$ and $R^6$ independently of one another are each fluorine, chlorine or bromine.

2. A process for the preparation of an N-benzoyl-N'-phenoxyphenylurea of the formula I as claimed in claim 1, wherein either a corresponding compound of the formula

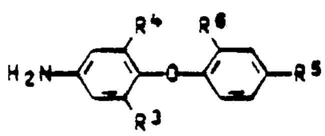

(II)

is reacted with a corresponding benzoyl isocyanate of the formula

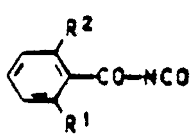

(III)

or a corresponding compound of the formula

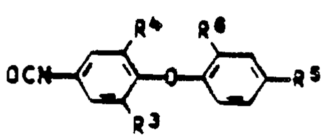

(IV)

is reacted with a corresponding benzamide of the formula

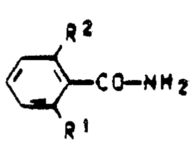

(V).

3. A pesticide containing an N-benzoyl-N'-phenoxyphenylurea of the formula I as claimed in claim 1.

4. A pesticide containing a solid or liquid carrier and/or a synergistic agent and an N-benzoyl-N'-phenoxyphenylurea of the formula I as claimed in claim 1.

5. Use of an N-benzoyl-N'-phenoxyphenylurea of the formula I as claimed in claim 1 for controlling pests.

6. A method for controlling pests, wherein an effective amount of an N-benzoyl-N'-phenoxyphenylurea of the formula I as claimed in claim 1 is allowed to act on the pests and/or their habitat.

## Revendications

1. N-benzoyl-N'-phénoxy-phénylurée de formule

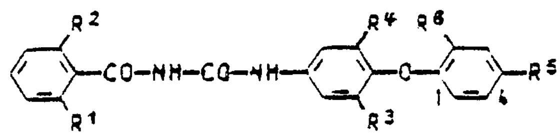

(I),

dans laquelle
$R^1$ représente fluor ou chlore
$R^2$ fluor, chlore (ou hydrogène, si $R^1$ est chlore)
$R^3$ et $R^4$ indépendamment,chlore ou brome et
$R^5$ et $R^6$ indépendamment, fluor, chlore ou brome.

2. Procédé de préparation de N-benzoyl-N'-phénoxy-phénylurées de formule 1 selon la revendication 1, caractérisé par le fait que l'on fait réagir
- soit un composé correspondant de formule

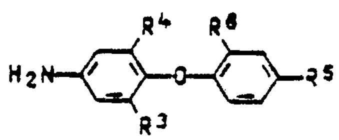

(II),

avec un isocyanate de benzoyle de formule

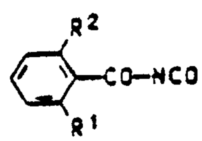

(III),

- soit un composé correspondant de formule

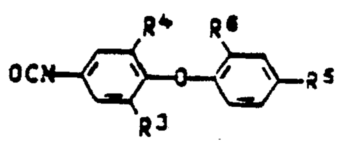

(IV)

avec un benzamide correspondant de formule

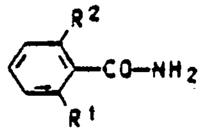

(V)

3. Produit anti-parasitaire contenant un N-benzoyl-N'-phénoxy phénylurée de formule I selon la revendication 1.

4. Produit anti-parasitaire contenant un support solide ou liquide et/ou un synergiste et une N-benzoyl-N'-phénoxy phénylurée de formule I selon la revendication 1.

5. Utilisation de N-benzoyl-N'-phénoxy phénylurées de formule I selon la revendication 1 pour lutter contre les parasites.

6. Procédé pour lutter contre les parasites, caractérisé par le fait que l'on fait agir sur les parasites et/ou leur biotope une quantité efficace d'un N-bnezoyl-N'-phénoxy phénylurée de formule I selon la revendication 1.